(19) Europäisches Patentamt
European Patent Office
Office européen des brevets

(11) Veröffentlichungsnummer: **0 075 817**
**B1**

(12) # EUROPÄISCHE PATENTSCHRIFT

(45) Veröffentlichungstag der Patentschrift: **12.03.86**

(51) Int. Cl.[4]: **B 01 D 3/38** // A61K7/46

(21) Anmeldenummer: **82108645.1**

(22) Anmeldetag: **18.09.82**

(54) **Verfahren zur destillativen Trennung von flüssigen, Hydrierungsprodukte des Citrals enthaltenden Stoffgemischen.**

(30) Priorität: **26.09.81 DE 3138423**

(43) Veröffentlichungstag der Anmeldung: **06.04.83 Patentblatt 83/14**

(45) Bekanntmachung des Hinweises auf die Patenterteilung: **12.03.86 Patentblatt 86/11**

(84) Benannte Vertragsstaaten: **CH DE FR GB LI NL**

(56) Entgegenhaltungen:
**DE - B - 2 323 420**
**DE - C - 737 621**
**US - A - 3 347 681**
**US - A - 3 787 593**

(73) Patentinhaber: **BASF Aktiengesellschaft, Carl-Bosch-Strasse 38, D-6700 Ludwigshafen (DE)**

(72) Erfinder: **Aquila, Werner, Dr., Meissener Weg 35, D-6800 Mannheim 42 (DE)**
Erfinder: **Nissen, Axel, Dr., Panoramastrasse 50, D-6906 Leimen (DE)**
Erfinder: **Kaibel, Gerd, Robert-Bosch-Strasse 4, D-6840 Lampertheim 1 (DE)**
Erfinder: **Horner, Michael, Dr., Im Ritterbueschel 9, D-6730 Neustadt (DE)**
Erfinder: **Rebafka, Walter, Dr., Lessingstrasse 4, D-6945 Hirschberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur destillativen Trennung von flüssigen Stoffgemischen aus

– Hydrierungsprodukten des Citrals und

– einem oder mehreren Stoffen, die niedriger als Wasser sieden und die mit Wasser keine Azeotrope bilden.

Bei der Trennung von Stoffmischungen durch Destillation ist die thermische Zersetzung des weniger flüchtigen Stoffes ein wesentliches Problem, weshalb man die Destillation vorzugsweise dann anwendet, wenn der oder die leichter flüchtigen (niedriger siedenden) Stoffe als Wertprodukt gewonnen werden sollen.

Der Fall, dass der oder die schwerer flüchtigen (höhersiedenden) Stoffe das (oder eines der) Wertprodukte darstellen, ist zwar an sich wohl ebenso häufig, jedoch wird in diesen Fällen auch meist versucht, den nach der Destillation zurückbleibenden Sumpf z.B. unter vermindertem Druck zu destillieren, wobei mitspielt, dass dieser Sumpf oft auch, ohne dass Zersetzung stattgefunden hat, Nebenprodukte etwa einer vorangegangenen Synthese enthält, die abgetrennt werden müssen.

Wie im Falle des vorliegenden Stoffgemisches kommt es jedoch auch vor, dass der oder die schwerflüchtigen Stoffe (hier: die Hydrierungsprodukte des Citrals) an sich sehr rein sind, jedoch sehr zur Zersetzung neigen und z.B. lediglich von einem oder mehreren Lösungsmitteln getrennt werden müssen.

In derartigen Fällen werden oft besondere Destillationseinrichtungen, z.B. Dünnschichtverdampfer angewendet, die eine schonende Stofftrennung ermöglichen sollen. Das Prinzip solcher Einrichtungen besteht in einem möglichst vollständigen Gas/Flüssig-Stoffaustausch in möglichst kurzer Zeit, d.h. einer Kombination von grossem Oberflächenangebot mit kurzer Verweilzeit.

Der Nachteil solcher Einrichtungen ist ihr geringes Trennvermögen, das rein theoretisch die Trennleistung eines Bodens nicht überschreiten kann sowie ihre begrenzte Kapazität, so dass sich technische Verfahren erheblich verteuern.

Aus der DE-AS 23 23 420 ist es ferner bekannt, flüssige Zweikomponenten-Gemische, z.B. Öl und Perchlorethylen, destillativ in ihre Komponenten zu zerlegen, indem man zunächst die leichter flüchtige Flüssigkeit unter allmählicher Temperaturerhöhung bis zu einem gewissen Grade abdestilliert und danach die Destillation nach Zusatz von emulgatorhaltigem Wasser fortsetzt. Bei diesem Verfahren werden Wasser und leichter flüchtige Komponenten (Perchlorethylen) gemeinsam verdampft und kondensiert, wonach das Wasser in einem Abscheider vom Perchlorethylen abgetrennt und wieder in die Destillation zurückgeführt wird. Dieses Verfahren ist jedoch wegen der zweistufigen Betriebsweise – erst ohne, dann mit Wasser – und der Kreisführung des Wassers technisch nachteilig und lässt sich überdies nur mit dem erhöhten Aufwand einer zusätzlichen destillativen Trennung des Kondensates in Wasser und leichter flüchtige Komponente auf den Fall übertragen, wenn die leichter flüchtige Komponente wasserlöslich ist.

Der Erfindung lag daher die Aufgabe zugrunde, die eingangs definierten Stoffgemische zur Gewinnung von reinen Hydrierungsprodukten des Citrals als den schwerflüchtigen Komponenten auf wirtschaftlichere Weise zu trennen, als nach den hierfür an sich bekannten Methoden.

Demgemäss wurde ein Verfahren zur destillativen Trennung von flüssigen Stoffgemischen aus

– Hydrierungsprodukten des Citrals und

– einem oder mehreren Stoffen, die niedriger als Wasser sieden und die mit Wasser keine Azeotrope bilden, gefunden, welches dadurch gekennzeichnet ist, dass man dem zu destillierenden Gemisch soviel Wasser zusetzt, dass der Siedepunkt des Wassers unter dem angewandten Druck die Sumpftemperatur des zu trennenden Stoffgemisches begrenzt und dass man die Destillation so gestaltet, dass keine nennenswerten Mengen Wasser in das Destillat gelangen.

Weiterhin wurde gefunden, dass es hierbei zweckmässig ist, die Stoffmischung bzw. den oder die zurückbleibenden Stoffe und das Wasser mit mechanischen oder chemischen Mitteln zu emulgieren, und dass das Verfahren besonders erfolgreich ist, wenn es sich bei den definitionsgemässen Stoffen, die niedriger als Wasser sieden, um Methanol und/oder Trimethylamin handelt.

Auf die Menge des Wassers kommt es an sich nicht an, sondern nur darauf, dass die Sumpftemperatur nicht über die Siedetemperatur des Wassers ansteigen kann. Hierfür genügen theoretisch wenige Tropfen.

Praktisch ist allerdings wünschenswert, dass keine örtliche Überhitzung auftreten kann; es wird deshalb angestrebt, dass möglichst überall im Bereich des Verdampfungsgefässes Wasser zugegen ist. Am besten erreicht man dies, indem man das Wasser mit dem schwersiedenden Teil der Mischung emulgiert. In den Fällen, wo zunächst ein einheitliches Gemisch vorliegt (z.B. wenn der leichtsiedende Bestandteil ein Alkohol o.ä. ist) tritt bei der Entmischung im Masse des Absiedens ohnehin meist eine Emulsion auf; sonst kann man eine ausreichende Verteilung auch durch Rühren u.ä. bewirken.

Bei den Hydrierungsprodukten des Citrals (3,7-Dimethylocta-2,6-dienal)

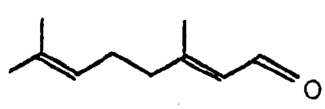

handelt es sich um eine Reihe wichtiger Riechstoffe. Mit palladiumhaltigen Katalysatoren lässt sich z.B. durch Hydrieren einer Doppelbindung Citronellal gewinnen. Aus diesem erhält man durch Hydrierung der Carbonylgruppe mit rutheniumhaltigen Katalysatoren Citronellol. Bei alleiniger Hydrierung der Carbonylgruppe bildet sich aus trans-Citral Geraniol und aus cis-Citral Nerol.

Im Hinblick auf die besonders bei Riechstoffen erforderliche hohe Reinheit ist es wichtig, bei der Synthese einen möglichst vollständigen Umsatz zu jeweils einem bestimmten Produkt zu erzielen, um die Operation der Trennung von Ausgangsstoff und Zielprodukt von vorneherein zu vermeiden. Gleichzeitig muss natürlich die Hydrierung sehr selektiv erfolgen, damit jeweils möglichst geringe Mengen an Nebenprodukten gebildet werden. Die genannten Stoffe lassen sich nämlich voneinander nur schlecht trennen, da die Siedepunkte in der Gegend um 220 °C dicht beieinander liegen. Andere Methoden, z.B. Extraktionsverfahren, sind nicht bekannt.

Um einen vollständigen Umsatz zu erzielen, arbeitet man nach einem Vorschlag in der DE-OS 28 39 474 bei diesen Hydrierungen stets mit einem Lösungsmittel, und zwar einem Zusatz eines Gemisches aus Methanol und z.B. Trimethylamin. Der Amingehalt in dem Lösungsmittelgemisch beträgt ca. 10 bis 50 Gew.-% und muss natürlich aus dem Zielprodukt wieder vollständig entfernt werden. Man destilliert zweckmässig zunächst bei atmosphärischem oder nur leicht vermindertem Druck, um eine hinreichend hohe Kondensationstemperatur zu erreichen.

Um hierbei das Wertprodukt völlig lösungsmittelfrei zu gewinnen, müsste die maximal zulässige Sumpftemperatur, die für Citronellal bei etwa 90–110 °C und für Terpenalkohole bei ca. 100 bis 130 °C liegt, weit überschritten werden, so dass man Zersetzungsprodukte erhielte. Die Bildung solcher Nebenprodukte lässt sich also nur durch Einhaltung der angegebenen Temperaturobergrenzen vermeiden, wobei im jeweiligen Wertprodukt jeweils noch etwa 10% an Lösungsmittel verbleibt.

Dieser Lösungsmittelrest wurde bisher bei einem Druck von etwa 2–15 mbar zusammen mit gewissen leichter siedenden Nebenprodukten abgetrennt. Trimethylamin z.B. lässt sich unter diesen Bedingungen jedoch auch mit Sole nicht mehr kondensieren, sondern muss über die Vakuumpumpe abgeführt und aus deren Abgasen z.B. ausgewaschen oder sonst unwirksam gemacht werden. Abgesehen vom Lösungsmittelverlust treten aber bei kontinuierlichem Betrieb auch noch Störungen auf, die mit der Temperaturregelung von Destillationskolonnen zusammenhängen und somit eine Abkehr von diesem Verfahren wünschenswert erscheinen lassen.

Erfindungsgemäss lassen sich die Folgeprodukte der Hydrierung von Citral von Methanol und/oder Trimethylamin und ggf. auch anderen vergleichbaren Stoffen einfach durch Zusatz von Wasser bei der Destillation trennen, ohne dass eine relativ hohe Temperatur benötigt wird.

Mann muss lediglich im Kolonnensumpf für eine ausreichende Emulgierung sorgen. Wasser ist geruchlich neutral und hat einen genügend niedrigen Siedepunkt. Da die Stoffkosten für Wasser nicht ins Gewicht fallen, ist es auch unerheblich, wenn es bei der Reindestillation verloren geht. Methanol und Trimethylamin lassen sich ohne Azeotropbildung vom Wasser abtrennen.

Da das Wasser nicht übergetrieben wird, sondern nur regulierend auf die höchste Temperatur des Gemisches wirkt, handelt es sich, dies sei ausdrücklich wiederholt, nicht um eine Wasserdampfdestillation. Selbst wenn ein schwersiedender Stoff mit Wasserdampf flüchtig sein sollte, ist das Prinzip anwendbar, da er in diesem Falle im Kolonnenteil der Anlage kondensiert und zurückgetrieben würde.

Ein scheinbarer Nachteil ist zunächst darin zu sehen, dass Wasser mit den lösungsmittelfreien Riechstoffen kaum mischbar ist. Die organische Phase im Verdampfer enthält weniger als ca. 0,3 Gew.-% Wasser und könnte an den Heizflächen eines normalen Verdampfers überhitzt und dadurch in der geruchlichen Qualität geschädigt werden. Dies wird jedoch vermieden, wenn man den Wasseranteil in der Verdampferflüssigkeit (Sumpf) auf mindestens 10 (z.B. bis 40) Gew.-% hält und aus Wasser und Produktgemisch eine Emulsion, d.h. ein zweiphasiges inniges Gemisch bildet, damit an den Heizflächen keine wasserarmen Zonen entstehen können. Dadurch bleibt die Sumpftemperatur auf etwa 100 °C begrenzt.

Die Emulgierung (Dispergierung) kann wie üblich durch Rührer oder vorzugsweise über eine externe Mischstrecke mittels einer Pumpe und einer Düse bewirkt werden (s. Figur).

Die Wasserzugabe kann direkt in den Kolonnensumpf erfolgen. Es ist aber auch möglich, das Wasser bei kontinuierlichem Betrieb in die Kolonne, z.B. zusammen mit dem Zulaufgemisch, zuzugeben.

Das zweiphasige, von Methanol und Trimethylamin freie Sumpfgemisch sammelt man in einem Trenngefäss. Die organische Phase, die einen konstanten Wassergehalt von etwa 0,3 Gew.-% Wasser hat, unterwirft man der Destillation unter vermindertem Druck, während die wässrige Phase zur Lösungsmitteldestillation zurückgeführt wird. Da sich in der organischen Phase nur wenig Wasser löst, müssen bei der Lösungsmitteldestillation nur diese geringen Mengen ergänzt werden.

Beispiel 1

- hierzu Fig.

Eine bei Normaldruck betriebene 4 m hohe mit Metall-Raschigringen mit 8 mm Durchmesser gefüllte Glaskolonne (1) wurde in Kolonnenmitte stündlich mit einem Gemisch aus 7,5 kg Citronellal, 5 kg Methanol und 2,5 kg Trimethylamin beschickt (2). Bei einem Rücklaufverhältnis von 2 wurden bei einer Kopftemperatur von 40 °C Methanol und Trimethylamin vollständig zusammen mit etwa 0,04 kg/h Wasser über Kopf abgetrennt. Zum Kolonnensumpf (102 °C) wurden stündlich 0,2 kg Wasser zugegeben (3). Hierdurch entstand eine zweiphasige Sumpfflüssigkeit, die etwa 20 Gew.-% Wasser enthielt.

Die Kolonne wurde über einem Umlaufverdampfer (4) mit 0,4 $m^2$ Heizfläche beheizt. Der Flüssigkeitszwangsumlauf wurde durch eine Kreiselpumpe (5) mit einer Umwälzleistung von ca. 1,5 $m^3/h$ bewirkt.

Das Sumpfprodukt wurde auf etwa 70 °C gekühlt und in ein Trenngefäss (6) geleitet. Die organische Phase wurde entnommen und die wässrige Unterphase in den Kolonnensumpf zurückgepumpt.

Beispiel 2

In der im Beispiel 1 beschriebenen Anlage wurden stündlich bei Normaldruck 10 kg eines Gemisches, das aus 50% Citronellol, 35% Methanol und 15% Trimethylamin bestand, kontinuierlich getrennt. Bei einem Rücklaufverhältnis von v = 2 wurde bei einer Kopftemperatur von 45 °C Methanol und Trimethylamin mit einem Gehalt von unter 0,5% $H_2O$ abgenommen.

Zum Kolonnensumpf wurden 0,2 kg Wasser/h zugegeben, wobei sich eine Sumpftemperatur von 95 °C einstellte.

Der aus dem Sumpf abgezogene zweiphasige Sumpfaustrag war aminfrei.

Beispiel 3

In der beschriebenen Anlage wurden 10 kg eines Gemisches aus Citral, Methanol, Trimethylamin (50%, 35%, 15%) bei Normaldruck getrennt.

Bei einem Rücklaufverhältnis von v = 0,5 bei einer Übergangstemperatur von 40 °C wurden Trimethylamin, Methanol und ca. 0,8% $H_2O$ abgezogen.

In den Sumpf der Kolonne wurde stündlich 0,5 l $H_2O$ zudosiert und damit die Sumpftemperatur auf 100 °C begrenzt.

Der zweiphasige Sumpfaustrag enthielt nur noch Spuren an Trimethylamin.

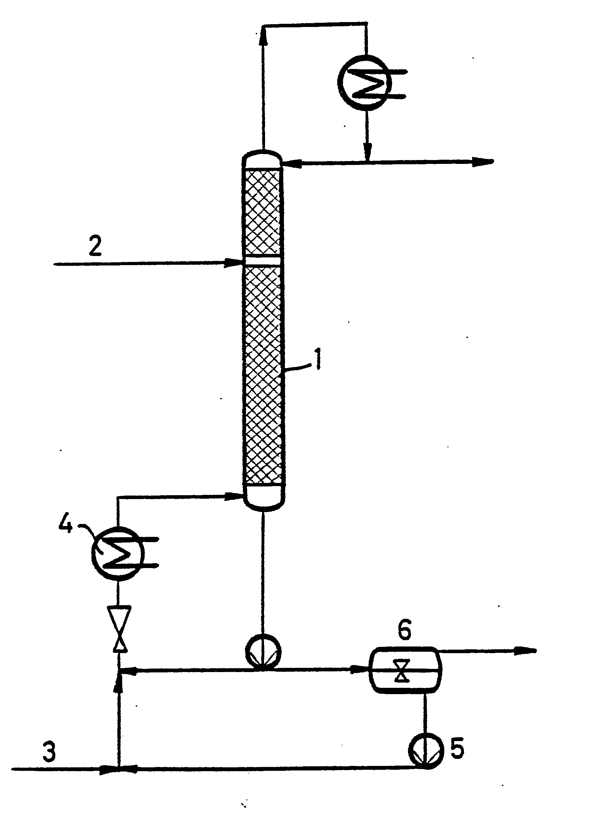

**Patentansprüche**

1. Verfahren zur destillativen Trennung von flüssigen Stoffgemischen aus

– Hydrierungsprodukten des Citrals und

– einem oder mehreren Stoffen, die niedriger als Wasser sieden und die mit Wasser keine Azeotrope bilden, dadurch gekennzeichnet, dass man dem zu destillierenden Gemisch soviel Wasser zusetzt, dass der Siedepunkt des Wassers unter dem angewandten Druck die Sumpftemperatur des zu trennenden Stoffgemisches begrenzt und dass man die Destillation so gestaltet, dass keine nennenswerten Mengen Wasser in das Destillat gelangen.

2. Verfahren nach Anspruch 1, dadurch gekennzeichnet, dass man die Stoffmischung bzw. den oder die zurückbleibenden Stoffe und das Wasser mit mechanischen oder chemischen Mitteln emulgiert.

3. Verfahren nach den Ansprüchen 1 und 2, dadurch gekennzeichnet, dass es sich bei den definitionsgemässen Stoffen, die niedriger als Wasser sieden, um Methanol und/oder Trimethylamin handelt.

**Revendications**

1. Procédé pour la séparation par distillation de mélanges liquides se composant

– de produits d'hydrogénation du citral et

– d'une ou de plusieurs substances dont le point d'ébullition est plus bas que celui de l'eau et qui ne forment pas d'azéotrope avec l'eau, caractérisé en ce qu'on ajoute, au mélange à distiller, suffisamment d'eau pour que le point d'ébullition de l'eau, sous la pression appliquée, limite la température de bas de colonne du mélange de substances à séparer, et en ce qu'on mène la distillation de telle manière qu'il ne parvienne pas de quantités appréciables d'eau dans le distillat.

2. Procédé selon la revendication 1, caractérisé en ce qu'on émulsionne le mélange de substances ou la ou les substances résiduelles et l'eau par des moyens mécaniques ou chimiques.

3. Procédé selon la revendication 1 ou 2, caractérisé en ce qu'il s'agit, pour ce qui est des substances selon la définition ci-dessus dont le point d'ébullition est plus bas que celui de l'eau, de méthanol et/ou de triméthylamine.

**Claims**

1. A process for the distillative separation of liquid mixtures of substances, consisting of

– hydrogenation products of citral, and

– one or more substances which have boiling points lower than that of water and do not form azeotropes with water, wherein such a quantity of water is added to the mixture to be distilled that the boiling point of water, under the pressure applied, limits the bottom temperature of the mixture of substances to be separated, and the distillation is so conducted that appreciable amounts of water do not enter the distillate.

2. A process as claimed in claim 1, wherein the mixture of substances or the substance or substances which remain and the water are emulsified by mechanical or chemical means.

3. A process as claimed in claims 1 and 2, wherein the said substances which have boiling points lower than that of water are methanol and/or trimethylamine.

1/1